# EUROPÄISCHE PATENTSCHRIFT

(11) **EP 2 066 401 B1**
(45) Veröffentlichungstag und Bekanntmachung des Hinweises auf die Patenterteilung: **05.12.2012**
(21) Anmeldenummer: 07802327.2
(22) Anmeldetag: 15.09.2007
(51) Int. Cl.: A61N 1/36

(54) **IMPLANTIERBARE VORRICHTUNG**
IMPLANTABLE DEVICE
DISPOSITIF IMPLANTABLE

(30) Priorität: 26.09.2006 DE 102006047117
(43) Veröffentlichungstag der Anmeldung: 10.06.2009
(73) Patentinhaber: Retina Implant AG, 72770 Reutlingen (DE)
(72) Erfinder: ROTHERMEL, Albrecht, 89231 Neu-Ulm (DE)
(74) Vertreter: Witte, Weller & Partner
(86) Internationale Anmeldenummer: PCT/EP2007/008043
(87) Internationale Veröffentlichungsnummer: WO 2008/037362

(56) Entgegenhaltungen:
- EP-A- 1 666 002
- US-A1- 2004 181 265

## Beschreibung

Die vorliegende Erfindung betrifft eine Vorrichtung mit einer Versorgungseinheit und einem mit der Versorgungseinheit verbundenen, implantierbaren Implantat, das dazu dient, über zumindest eine Stimulationselektrode elektrische Stimulationssignale an umgebendes Gewebe abzugeben, wobei das Implantat über seine mit der Versorgungseinheit verbundene Eingangsstufe mit elektrischer Energie versorgt wird.

Derartige Vorrichtungen werden heute vielfach verwendet, um bestimmte physiologische Funktionen des bspw. menschlichen Körpers zu unterstützen oder zu ersetzen, oder um Sinneswahrnehmungen zu unterstützen bspw. erst wieder möglich zu machen. Zu diesen Vorrichtungen zählen beispielhaft aber nicht abschließend Herzschrittmacher, Cochlear-Implantate oder Retina-Implantate.

Aktive Retina-Implantate zur Implantation ins Auge sind beispielsweise mit einer Vielzahl von Stimulationselektroden versehen, die elektrische Stimulationssignale an zu kontaktierende Zellen der Retina abgeben. Eine Vielzahl von Bildelementen wandelt dabei einfallendes Licht in die Stimulationssignale um.

Ein derartiges Retina-Implantat ist beispielsweise aus der WO 2005/000395 A1 bekannt, deren Offenbarung hiermit ausdrücklich in die vorliegende Anmeldung einbezogen wird.

Das bekannte Retina-Implantat dient dazu, einem Verlust des Sehvermögens aufgrund von Retina-Degenerationen entgegenzuwirken. Grundgedanke ist es dabei, einem Patienten einen mikroelektronischen Stimulationschip in das Auge zu implantieren, der durch elektrische Anregung von Nervenzellen das verloren gegangene Sehvermögen ersetzt.

Dabei gibt es zwei unterschiedliche Ansätze, wie derartige Retina-Prothesen ausgelegt sein können. Der subretinale Ansatz verwendet einen in den subretinalen Raum zwischen die äußere Retina und das Pigmentepithel der Retina implantierten Stimulationschip, der auf ein in den Stimulationschip integriertes Array von Photodioden auffallendes Umgebungslicht in Stimulationssignale für Nervenzellen umsetzt. Dieses Retina-Implantat stimuliert also die verbleibenden, intakten Neuronen der degenerierten Retina, also Horizontalzellen, Bipolarzellen, Amakrinzellen und möglicherweise auch Ganglionzellen.

Das auf das Array von Photodioden oder komplexeren Elementen auftreffende visuelle Bild wird also in ein elektrisches Stimulationsmuster umgewandelt, das dann von dem "natürlichen Computer" zu den Ganglionzellen der inneren Retina geleitet und von dort über den Sehnerv in den visuellen Cortex geführt wird. Mit anderen Worten, der subretinale Ansatz nutzt die natürliche Verschaltung der ehemals vorhandenen und jetzt degenerierten oder verloren gegangenen Photorezeptoren mit den Ganglionzellen aus, um dem visuellen Cortex in gewohnter Weise Nervenimpulse zuzuführen, die dem gesehenen Bild entsprechen.

Im Gegensatz dazu nutzt der epiretinale Ansatz eine aus einem extra-okularen und einem intra-okularen Teil bestehende Vorrichtung, die auf geeignete Weise miteinander kommunizieren. Das extra-okulare Teil umfasst eine Kamera und eine mikroelektronische Schaltung, um aufgefangenes Licht, also die Bildinformation, zu decodieren und als Stimulationsmuster an das intra-okulare Teil zu übertragen. Das intra-okulare Teil enthält ein Elektrodenarray, das Neuronen der inneren Retina kontaktiert und so die dort befindlichen Ganglionzellen unmittelbar stimuliert.

Während der subretinale Ansatz die Übertragung von Licht und die Stimulation der Retina *in situ* verfolgt, müssen bei dem epiretinalen Ansatz die Bildinformationen extern in ein räumliches und zeitliches Stimulationsmuster von elektrischen Pulsen umgewandelt werden, damit sie von dem visuellen Cortex "verstanden" werden können.

Aus vielerlei Veröffentlichungen ist es bekannt, dass die Übertragung der Stimulationssignale von den Stimulationselektroden zu den kontaktierten Zellen besonderer Aufmerksamkeit bedarf. Die Kopplung zwischen einer Stimulationselektrode und dem kontaktierten Gewebe ist nämlich kapazitiver Natur, so dass zur Stimulation nur transiente Signale verwendet werden können. Diese kapazitive Kopplung beruht darauf, dass sich an der Grenzfläche zwischen Elektrode und Elektrolyt im Auge eine Kapazität (Helmholtz-Doppelschicht) infolge der Elektrodenpolarisation ausbildet.

Bei dem subretinalen Implantat gemäß der eingangs erwähnten WO 2005/000395 wird das auffallende Licht daher in monophasische, anodische Spannungspulse mit einer Pulslänge von ca. 500 Mikrosekunden und einem Pulsabstand von vorzugsweise 50 msec umgewandelt, so dass sich eine Wiederholfrequenz von 20 Hz ergibt, die sich als ausreichend für flimmerfreies Sehen herausgestellt hat; sie entspricht auch der physiologischen Flimmerfrequenz bei niedriger Umgebungshelligkeit.

Der Pulsabstand im Bereich von 50 msec ist dabei ausreichend, um die Elektrodenpolarisation zurückführen zu können. Nach Abgabe des durch den jeweiligen anodischen Spannungspuls in das Gewebe geleiteten Stimulationsstroms wird der Ausgang des Implantates dazu durch eine Kurzschlussschaltung mit der elektrischen Masse des Implantates verbunden, so dass sich die Kapazität der Helmholtz-Doppelschicht wieder entlädt und im zeitlichen Mittel nahezu kein Ladungstransport in das Gewebe erfolgt.

Humayun et al., "Pattern Electrical Stimulation of the Human Retina", Vision Research 39 (1999) 2569-2576 berichten über Experimente mit epiretinaler Stimulation, bei der sog. biphasische Pulse verwendet werden, die eine kathodische Phase, eine Zwischenphase und eine anodische Phase von jeweils 2 Millisekunden aufweisen. Bei einer Stimulationsfrequenz zwischen 40 und 50 Hz, also deutlich oberhalb der physiologischen Flimmerfrequenz, konnte bei zwei Patienten eine flimmerfreie Wahrnehmung beobachtet werden.

Jensen et al., "Responses of Rabbit Retinal Ganglion Cells to Electrical Stimulation with an Epiretinal Electrode", J. Neural Eng. 2 (2005) 16-21, berichten über die epiretinale Anregung von Ganglienzellen bei einem Kaninchen. Bei anodischen und katodischen Strompulsen von 1 Millisekunde Länge beobachten sie zur Anregung auf der inneren Retina mittlere Latenzzeiten der Ganglionzellen zwischen 11 und 25 Millisekunden.

Jensen und Rizzo, "Thresholds for Activation of Rabbit Retinal Ganglion Cells with a Subretinal Electrode", Experimental Eye Research 2006, 1-7, berichten über subretinale Stimulationsexperimente an einer isolierten Kaninchen-Retina mit monophasischen Strompulsen von 0,1 Millisekunden bis 50 Millisekunden Länge, für die sie Latenzzeiten von ungefähr 25 Millisekunden beobachten.

Die Energie zur Erzeugung der elektrischen Stimulationssignale kann nun aber auch bei subretinalen Implantaten nicht aus dem einfallenden Nutzlicht selbst gewonnen werden, so dass zusätzlich Fremdenergie benötigt wird. Während bei Herzschrittmachern bereits seit längerem kabellose Implantate zur Verfügung stehen, die über eine mit-implantierte Batterie versorgt werden, ist wegen der kleineren Abmaße vieler anderer Implantate sowie der physiologischen Beschränkungen bspw. sowohl bei Retina- als auch bei Cochlear-Implantaten die permanente externe Zufuhr von Energie erforderlich.

Diese Fremdenergie wird bei Retina-Implantaten entweder durch zusätzlich eingestrahltes nicht-sichtbares Licht eingespeist oder extern beispielsweise über eine Spule induktiv eingekoppelt oder über ein Kabel zugeführt.

Das aus der WO 2005/000395 A1 bekannte Implantat wird daher über eingestrahltes IR-Licht oder über induktiv eingekoppelte HF-Energie kabellos mit elektrischer Energie versorgt, wobei in dieser extern zugeführten Fremdenergie Informationen zur Steuerung des Implantates enthalten sein können.

Aus der US 2004/0181265 A1 ist ein nur mit Umgebungslicht betriebenes Retina-Implantat bekannt, das weder für die Energieversorgung noch für die Bereitstellung von Bildinformationen externe Komponenten verwendet. Das bekannte Implantat umfasst ein implantiertes Feld von auf Umgebungslicht ansprechenden photovoltaische Zellen, die eine Versorgungsspannung für einen ebenfalls implantierten Stimulationschip erzeugen, über den sie mit einem im Auge verlaufenden Kabel verbunden sind.

Der Stimulationschip umfasst eine Anzahl von Pixeln, die jeweils eine lichtempfindliche Schaltung sowie eine damit verbundene Elektrode aufweisen. Über diese Elektrode werden Retina-Zellen mit biphasischen Pulsen stimuliert, die eine Pulsdauer von 1 ms sowie eine Wiederholrate von 25 Hz aufweisen. Die biphasischen Pulse sollen so ausgelegt sein, dass sie im zeitlichen Mittel keine Ladung in das umgebende Gewebe transportieren.

Da kabellose Retina-Implantate für Anwendungen am Menschen jedoch noch nicht mit einer zufriedenstellenden Qualität zur Verfügung stehen, werden zurzeit nicht nur epiretinale sondern auch subretinale Implantate verwendet, denen die erforderliche Fremdenergie über Kabel zugeführt wird.

Gekeler et al.: "Compound subretinal prostheses with extra-ocular parts designed for human trials: successful long-term implantation in pigs", Graefe's Arch Clin Exp Ophthalmol (28. April 2006) [Epub ahead of print], beschreiben bspw. ein subretinales Retina-Implantat, bei dem die Fremdenergie und Steuersignale kabelgebunden zu dem in das Auge implantierten Chip geleitet werden.

Da zum einen auf den Implantaten in der Regel integrierte Schaltkreise vorhanden sind, die mit Gleichspannung betrieben werden, und zum anderen auf den Implantaten selbst wenig Platz zur Verfügung steht, werden die bekannten Implantate unmittelbar mit Gleichspannung versorgt. Bei einer Versorgung mit Wechselspannung würden die auf dem Implantat erforderlichen Gleichrichter insbesondere wegen der benötigten Glättungskondensatoren zu viel Platz beanspruchen bzw. sich in integrierten Schaltungen auch technisch nicht sinnvoll realisieren lassen. Diese Probleme treten insbesondere bei subretinalen Retina-Implantaten auf, aber auch andere Implantate müssen natürlich platzsparend aufgebaut sein.

Die kabelgebundene Übertragung von Gleichspannung führt jedoch langfristig zu elektrolytischen Zersetzungsprozessen in dem die Kabel umgebenden Gewebe, so dass auch diese Art der Versorgung von Implantaten mit Fremdenergie nicht zufriedenstellend ist.

Die EP 1 666 002 A1 beschreibt einen abstimmbaren Resonanzkreis, der für die induktive Spannungsversorgung von medizinischen Implantaten vorgesehen ist. In der Schaltung können rechteckförmige Ausgangsspannungen verwendet werden.

Vor diesem Hintergrund liegt der vorliegenden Erfindung die Aufgabe zugrunde, die Versorgung der eingangs genannten Vorrichtungen mit elektrischer Energie auf vorzugsweise schaltungstechnisch einfache Weise zu verbessern.

Erfindungsgemäß wird diese Aufgabe bei der eingangs erwähnten Vorrichtung dadurch gelöst, dass die Versorgungseinheit mit dem Implantat über Kabel verbunden ist, über die sie die Eingangsstufe mit zumindest zwei im Wesentlichen rechteckförmigen elektrischen Wechselspannung vorsorgt, die gegenüber einer mit dem Gewebe verbindbaren externen Masse im zeitlichen Mittel zumindest nahezu gleichspannungsfrei sind, wobei die Wechselspannungen zueinander in der Phase verschoben sind.

Die der Erfindung zugrunde liegende Aufgabe wird auf diese Weise vollkommen gelöst.

Die Erfinder der vorliegenden Anmeldung haben nämlich erkannt, dass es weder erforderlich ist, das Implantat mit Gleichspannung zu versorgen, noch dass eine Wechselspannungsversorgung mit entsprechender Gleichrichtung auf dem Implantat erforderlich ist. Wenn vielmehr zwei im Wesentlichen rechteckförmige elektrische Wechselspannung als Versorgung eingespeist werden, so kann die Potentiallage so gewählt werden, dass die Versorgungsspannung im zeitlichen Mittel zumindest nahezu gleichspannungsfrei ist. Auf diese Weise werden die störenden elektrolytischen Zersetzungsprozesse zumindest größtenteils vermieden.

Auf dem Implantat, das im Folgenden auch als Chip bezeichnet wird, steht daher eine rechteckförmige Wechselspannung zur Verfügung, die jetzt in eine Versorgungsgleichspannung umgewandelt werden muss. Im einfachsten Fall ist dies schon dadurch möglich, dass lediglich die positiven Pulse oder "Halbwellen" als Versorgungsgleichspannung verwendet werden, es entsteht dann eine Art pulsierende Gleichspannung, die jedoch nicht über Kondensatoren geglättet werden muss. Es ist beispielsweise möglich, bei entsprechend längeren Pulsdauern von mehreren hundert Millisekunden die Schaltungen auf dem Chip synchron zu den positiven Spannungspulsen jeweils abzuschalten. Andererseits ist es möglich, durch einfache Umschalter oder Umrichter dafür zu sorgen, dass auch die negativen Halbwellen der externen rechteckförmigen Wechselspannung sozusagen nach oben geklappt werden, wie dies beispielsweise auch mit Brückengleichrichtem möglich ist.

Die Versorgungsgleichspannung besteht dann zwar immer noch aus Spannungspulsen, zwischen denen wegen der endlichen Flankensteilheit bei den Polaritätswechseln kleine Spannungslücken auftreten, eine derartige gepulste Versorgungsgleichspannung kann jedoch insbesondere dann zur elektrischen Versorgung der Implantate verwendet werden, wenn - wie bereits erwähnt - während der Spannungslücken oder -einbrüche die Elektronik jeweils abgeschaltet wird, was am einfachsten dadurch erreicht wird, dass die Versorgungsgleichspannung am Ausgang des entsprechenden Gleichrichters jeweils abgeschaltet wird.

Mit anderen Worten, durch die erfindungsgemäße Verwendung einer nahezu rechteckförmigen Wechselspannung als elektrische Versorgung kann die Eingangsstufe platzsparend ausgelegt werden, da auf dem Chip keine Glättung oder Stabilisierung erforderlich ist.

Andererseits kann eine eventuell gewünschte Glättung bei Bedarf durch die auf dem Chip ohnehin vorhandenen parasitären Kapazitäten erreicht werden, wenn die Umschaltzeit kurz genug und die Stromaufnahme während dieser Zeit klein genug sind.

Weil die Eingangsstufe von der Versorgungseinheit erfindungsgemäß jedoch mit zumindest zwei im Wesentlichen rechteckförmigen elektrischen Wechselspannungen versorgt wird, können durch die Verschiebung in der Phase die Spannungseinbrüche oder Lücken bei der Gleichrichtung einer der beiden Wechselspannungen durch die andere Wechselspannung ausgeglichen werden . Auf schaltungstechnisch einfache Weise ist es dann ohne Verwendung von Kondensatoren möglich, eine konstante oder zumindest nahezu konstante Versorgungsgleichspannung bereitzustellen, wobei die kabelgebundene Zufuhr der elektrischen Energie im zeitlichen Mittel gleichspannungsfrei erfolgt.

Andererseits ist es bevorzugt, wenn die Eingangsstufe eine Gleichrichterschaltung umfasst, die die Wechselspannungen gleichrichtet und zu einer Versorgungsgleichspannung addiert.

Hier ist von Vorteil, dass die Amplituden der beiden rechteckförmigen Wechselspannungen selbst jeweils etwa nur halb so groß sein müssen, wie die tatsächlich benötigte Versorgungsgleichspannung. Wenn bspw. beide Versorgungsspannungen eine Pulsfolge von +1,5 V und -1,5 V gegenüber der externen Masse aufweisen, kann auf dem Chip eine gegenüber der dortigen elektrischen Masse bestehende Versorgungsgleichspannung von 3 V erzeugt werden.

Die geringeren Spannungshübe der beiden Wechselspannungen haben den weiteren Vorteil, dass zusätzlich zu der im zeitlichen Mittel gleichspannungsfreien Versorgung auch nur geringe Spannungen anliegen, die eine elektrolytische Zersetzung bewirken könnten.

Dabei ist es insbesondere bevorzugt, wenn die Eingangsstufe mit einer ersten und einer zweiten Wechselspannung versorgt wird, die bei etwa gleichem Kurvenverlauf und etwa gleichen Amplituden zumindest nahezu invers zueinander verlaufen.

Bei dieser Maßnahme ist von Vorteil, dass die Gleichrichterschaltung sehr einfach aufgebaut sein kann, da die extern angelieferte rechteckförmige Wechselspannung symmetrisch ist, so dass vergleichbare oder spiegelbildliche Schaltungen für die Gleichrichtung der beiden Wechselspannungen verwendet werden können.

Weiter ist es dann bevorzugt, wenn die Gleichrichterschaltung aus der ersten und der zweiten Wechselspannung die Versorgungsgleichspannung derart erzeugt, dass ihre elektrische Masse und die Versorgungsgleichspannung symmetrisch zu der externen Masse liegen.

Diese Maßnahme ermöglicht eine sehr einfache Gleichrichtung der extern zugeführten Spannungen, die negativen Pulse werden durch die Gleichrichterschaltung dann jeweils mit der elektrischen Masse und die positiven Pulse mit der Versorgungsgleichspannung verbunden. Die Symmetrie zu der externen Masse ergibt sich dann deshalb, weil die beiden rechteckförmigen Wechselspannungen jeweils symmetrisch zu der externen Masse liegen.

Ferner ermöglicht diese Maßnahme es, dass auf schaltungstechnisch einfache Weise die Stimulationssignale im zeitlichen Mittel ebenfalls gegenüber der externen Masse gleichspannungsfrei abgegeben werden können, denn auf dem Chip steht jetzt eine gegenüber der externen Masse - also dem Patienten negative - Spannung zur Verfügung.

In diesem Zusammenhang ist es dann bevorzugt, wenn die Gleichrichterschaltung Schalter für die jeweilige Wechselspannung aufweist, die über die jeweils andere Wechselspannung angesteuert werden.

Diese Maßnahme ermöglicht einen sehr einfachen Aufbau der Gleichrichterschaltung, die positiven Pulse beider Gleichspannungen werden entsprechend ihrem Versatz in der Phase zueinander jeweils zu der Versorgungsgleichspannung durchgeschaltet, indem die dann jeweils negativen Pulse der anderen Wechselspannung zur Ansteuerung der Schalter verwendet werden. Auf die gleiche Weise werden die jeweiligen negativen Pulse der Wechselspannungen zu der elektrischen Masse durchgeschaltet, indem die entsprechenden Schalter durch die positiven Pulse der jeweils anderen Wechselspannung angesteuert werden.

In diesem Zusammenhang ist es dann bevorzugt, wenn die Gleichrichterschaltung zwei Äste mit jeweils zwei an ihren Ausgangselektroden parallel geschalteten Feldeffekttransistoren umfasst, wobei die Ausgangselektroden in dem ersten Ast die elektrische Masse bilden und die Ausgangselektroden in dem zweiten Ast die Versorgungsgleichspannung abgeben, und wobei jeder Feldeffekttransistor an seiner Eingangselektrode mit einer der beiden Wechselspannungen und an seiner Steuerelektrode mit der anderen der beiden Wechselspannungen verbunden ist.

Diese Maßnahme ist eine technisch besonders einfache und sinnvolle Umsetzung der oben beschriebenen Gleichrichtung, bei der die eine Wechselspannung jeweils durch die andere Wechselspannung geschaltet wird. Als Schalter werden dabei Feldeffekttransistoren verwendet, die zum einen nur eine sehr geringe Leistungsaufnahme haben und zum anderen verglichen mit den üblichen Gleichrichterdioden eine sehr geringe Durchlassspannung aufweisen. Auf diese Weise ist es möglich, aus den beiden zueinander um 180° in der Phase verschobenen rechteckförmigen Wechselspannungen eine nahezu durchgehend gleichmäßige Versorgungsgleichspannung aufzubauen, lediglich an den Flankenübergängen der Pulse entstehen Spannungseinbrüche.

Um diesem Problem zu begegnen, ist es weiter bevorzugt, wenn der Ausgang des zweiten Astes über einen Schalter mit der Versorgungsgleichspannung verbunden ist, der immer dann geöffnet ist, wenn die Wechselspannungen ihre Polarität gegenüber der externen Masse ändern.

Auf diese Weise wird - wie schon oben erwähnt - eine konstante Versorgungsgleichspannung bereitgestellt, wobei die Dauer der Öffnung des Schalters, sehr kurz gehalten werden kann.

Die kleinen Spannungslücken können andererseits bspw. dadurch kompensiert werden, dass die Eingangsstufe mit einem zweiten Paar von zueinander inversen, im Wesentlichen rechteckförmigen Wechselspannungen versorgt wird, die gegenüber dem ersten Paar in der Phase verschoben ist, wobei diese Phasenverschiebung von 180° abweicht. Durch die Überlagerung der aus diesen beiden Paaren von Wechselspannungen erzeugten Versorgungsgleichspannungen kann dann eine zeitlich konstante Gleichspannung ohne Spannungseinbrüche erzeugt werden.

Es ist weiter bevorzugt, wenn die oder jede Wechselspannung einen trapezförmigen Spannungsverlauf aufweist, mit Dachphasen konstanter Spannung und kurzen Flankenphasen, in den die Polarität gegenüber der externen Masse wechselt, wobei die Länge der Dachphasen im Bereich von höchstens 100 msec und die Flankenphasen vorzugsweise höchstens 10 %, weiter vorzugsweise weniger als 1 % aber mehr als 0,05% der Dachphase betragen.

Bei dieser Maßnahme ist von Vorteil, dass nicht extrem steile Flanken verwendet werden, die bei entsprechender Gleichrichtung dann zu HF-Störungen führen könnten, und dass die Länge der Dachphasen abgestimmt ist auf die Wiederholfrequenz, mit der die Stimulationsimpulse aus physiologischen Gründen an das Gewebe abgegeben werden können.

Prinzipiell ist es zunächst so, dass beliebige Dachphasen, Flankenphasen und Wiederholfrequenzen möglich sind, rein aus elektronischer Sicht lassen sich auch erheblich kürzere oder erheblich längere Pulse verarbeiten als die hier relevanten Pulse zwischen 5 msec und 100 msec.

Insbesondere bei Retina-Implantaten sind jedoch die o.g. Größenordnungen sinnvoll, wie sich durch Experimente bestätigt hat.

Bei zwei informierten Patienten wurde nämlich von Wissenschaftlern der Anmelderin unter einem von der zuständigen Ethikkommission genehmigten Protokoll ein aktives Retina-Implantat der eingangs genannten Art subretinal implantiert und u.a. untersucht, welchen Einfluss verschiedene Wiederholfrequenzen und Pulslängen auf den visuellen Eindruck haben. Zu diesem Zweck enthielt das Implantat ein Raster von direkt zu stimulierenden Elektroden, die zueinander einen Abstand von 280 Mikrometern aufwiesen. Mit Hilfe einer externen Elektronik konnten Pulsform, Pulslänge und Pulswiederholfrequenz individuell eingestellt werden.

Die Netzhaut eines blinden Patienten wurde dabei subretinal über Elektroden mit biphasischen, anodisch beginnenden Pulsen von bis zu 4 msec Dauer gereizt. Bei Anwendung verschiedener Wiederholfrequenzen, also bei einer Anregung mit einer Dauerfolge von "Blitzen" bestimmter Frequenz, ergab sich folgende Beobachtung, was die Empfindung der Patienten betrifft:
Bei höheren Frequenzen etwa oberhalb von 10 Hz empfand der Patient nur eine kurze Zeitlang Blitze, danach verschwand die Wahrnehmung der Blitze subjektiv.
Bei einer elektrischen Reizung mit einer mittleren Frequenz unterhalb von 10 Hz wurden die Reizimpulse dagegen mindestens über einige Sekunden lang als getrennte Blitze wahrgenommen. Bei Frequenzen von wenigen Hz und darunter wurde dagegen jeder Blitz als Einzelblitz empfunden, die Empfindung blieb auch über Minuten hin stabil.

Wenn somit die Wiederholfrequenz der Pulse in den etwa rechteckförmigen Wechselspannungen der Wiederholfrequenz der Stimulationspulse entspricht, können Steuersignale unmittelbar aus der Wechselspannung abgeleitet werden.

In diesem Zusammenhang ist es dann bevorzugt, wenn das Implantat eine Sensoreinheit, die aus extern zugeführten Signalen ein Nutzsignal erzeugt, und eine Ausgangsstufe umfasst, die aus dem Nutzsignal Stimulationssignale erzeugt, wobei das Implantat vorzugsweise ein aktives Retina-Implantat ist, dessen Sensoreinheit eine Vielzahl von Bildzellen umfasst, die einfallendes Licht in elektrische Signale umwandelt, wobei die Sensoreinheit aus den elektrischen Signalen das Nutzsignal in Form von analogen Spannungspulsen bestimmter Pulslänge und bestimmter Pulsabstände erzeugt, deren Amplituden von der jeweiligen Intensität des einfallenden Lichtes ablenken.

Diese Maßnahme ist aus der eingangs erwähnten WO 2005/000395 A1 bereits bekannt, so dass wegen der damit verbundenen Vorteile auf diese Entgegenhaltung verwiesen werden darf.

Dabei ist es dann bevorzugt, wenn die Ausgangsstufe die Stimulationssignale im zeitlichen Mittel im Wesentlichen gleichspannungsfrei an umgebendes Gewebe abgibt, wozu sie vorzugsweise einen Wechselrichter umfasst.

Bei dieser Maßnahme ist von Vorteil, dass die elektrolytischen Zersetzungsprozesse in dem umgebenden Gewebe nicht nur im Bereich der die Versorgungsspannung liefernden Kabel, sondern auch im Bereich der Stimulationselektroden vermieden werden können. Der Wechselrichter stellt dabei eine schaltungstechnisch einfache Möglichkeit dar, die Spannungspulse umzupolen, zumal er über die Phasenwechsel der rechteckförmigen Wechselspannungen gesteuert werden kann.

Zwar ist es aus der eingangs erwähnten WO 2005/000395 A1 bereits bekannt, den Stimulationsstrom biphasisch auszulegen, so dass im Mittel kein Ladungstransport in das Gewebe erfolgt, bei dem bekannten Retina-Implantat erfolgt die Stimulation jedoch nicht im zeitlichen Mittel gleichspannungsfrei, wie dies jetzt erfindungsgemäß vorgesehen wird.

Dabei ist bevorzugt, wenn die Stimulationssignale durch den Wechselrichter als Folge von jeweils zwei unterschiedliche Phasen aufweisenden Pulsen an die Stimulationselektrode geliefert werden.

Bei dieser Maßnahme ist von Vorteil, dass auf schaltungstechnisch einfache Weise die Stimulationssignale im zeitlichen Mittel gegenüber der externen Masse gleichspannungsfrei abgegeben werden können, denn auf dem Chip steht jetzt eine gegenüber der externen Masse - also dem Patienten negative - Spannung zur Verfügung.

Der Wechselrichter stellt dabei eine schaltungstechnisch einfache Möglichkeit dar, die Spannungspulse umzupolen. Wegen der biphasischen, symmetrisch zu der externen Masse liegenden Stimulationssignale ergibt sich die Gleichspannungsfreiheit bereits über eine sehr kurzen Zeitspanne. Nur während dieser Zeitspanne liegen nacheinander Spannungen, die für eine elektrolytische Zersetzung bewirken könnten, wobei aber noch innerhalb der kurzen Zeitspanne des biphasischen Pulses ein inverser Spannungspuls abgegeben wird.

Insgesamt ist es dann bevorzugt, wenn der Wechselrichter die Spannungspulse synchron zu den Wechselspannungen in ihrer Polarität gegenüber der externen Masse umpolt.

Bei dieser Maßnahme ist von Vorteil, dass die sowieso vorhandene, von außen zugeführte Umpolung der externen Wechselspannungen gleichzeitig dazu verwendet werden kann, die Spannungspulse gegenüber der externen Masse umzupolen: Auf diese Weise ergeben sich gegenüber der externen Masse im zeitlichen Mittel gleichspannungsfreie Anregungsimpulse.

Es ist aber auch möglich, die Umpolung durch Steuersignale zu bewirken, die auf dem Chip generiert oder von außen zugeführt werden.

Dabei ist es bevorzugt, wenn der Wechselrichter zwei parallel zueinander zwischen den Ausgang der Sensoreinheit und die Stimulationselektrode geschaltete Äste aufweist, die beide an ihrem Ausgang je einen mit der ersten Wechselspannung verbundenen ersten Stromspiegel umfassen, wobei der eine Ast einen mit der zweiten Wechselspannung verbundenen zweiten Stromspiegel umfasst, der zwischen die Sensoreinheit und den ersten Stromspiegel geschaltet ist.

Diese Maßnahme ist insbesondere schaltungstechnisch von Vorteil, sie stellt eine elegante Lösung für die Umpolung der Spannungspulse dar, wobei sie gleichzeitig nur eine sehr geringe Stromaufnahme zeigt.

Weiter ist es bevorzugt, wenn die Sensoreinheit an ihrem Ausgang einen Ausgangsverstärker aufweist, dessen Ausgang über ein Steuersignal immer dann abgeschaltet wird, wenn die Wechselspannung ihre Polarität gegenüber der externen Masse ändert.

Auch diese Maßnahme ist schaltungstechnisch von Vorteil, denn der Ausgangspuls der Sensoreinheit wird sozusagen in zwei zu den Flanken der Wechselspannungen symmetrische Pulse umgewandelt, wobei jetzt lediglich noch einer der beiden Pulse in seiner Polarität geändert werden muss, um zu der im zeitlichen Mittel gleichspannungsfreien Abgabe von Anregungspulsen zu gelangen.

Schließlich ist es noch bevorzugt, wenn der Wechselrichter an seinem Ausgang einen Schalter aufweist, der den Ausgang immer dann kurzfristig mit der externen Masse verbindet, wenn ein Spannungspuls beendet ist, dessen Amplitude also für die Dauer des Pulsabstandes auf die interne Masse geschaltet wird.

Bei dieser Maßnahme ist von Vorteil, dass sich die durch den Spannungspuls aufgeladene Helmholtzkapazität an der Stimulationselektrode schnell wieder entlädt, wie dies prinzipiell auch bereits aus der eingangs erwähnten WO 2005/000395 A1 bekannt ist.

Weitere Vorteile ergeben sich aus der Beschreibung und der beigefügten Zeichnung.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ein Ausführungsbeispiel der Erfindung ist in der Zeichnung dargestellt und wird in der nachfolgenden Beschreibung näher erläutert. Es zeigen:
- Fig. 1: eine schematische Darstellung eines Retina-Implantats, in nicht maßstabsgetreuer Darstellung;
- Fig. 2: eine schematische Darstellung eines menschlichen Auges, in das das Retina-Implantat gemäß Fig. 1 eingesetzt ist, ebenfalls nicht maßstabgetreu;
- Fig. 3: eine schematische Darstellung des Retina-Implantates aus Fig. 1;
- Fig. 4: eine schematische Darstellung der Eingangsstufe des Retina-Implantates aus Figur 3;
- Fig. 5: eine schematische Darstellung der beiden Äste der Gleichrichterschaltung aus der Eingangsstufe aus Fig. 4;
- Fig. 6: eine schematische Darstellung der Sensoreinheit sowie der Ausgangsstufe des Retina-Implantates aus Fig. 3; und
- Fig. 7: eine schematische Darstellung verschiedener Steuersignale und Signalverläufe in der Eingangsstufe gemäß Fig. 4 sowie der Sensoreinheit und der Ausgangsstufe gemäß Fig. 6.

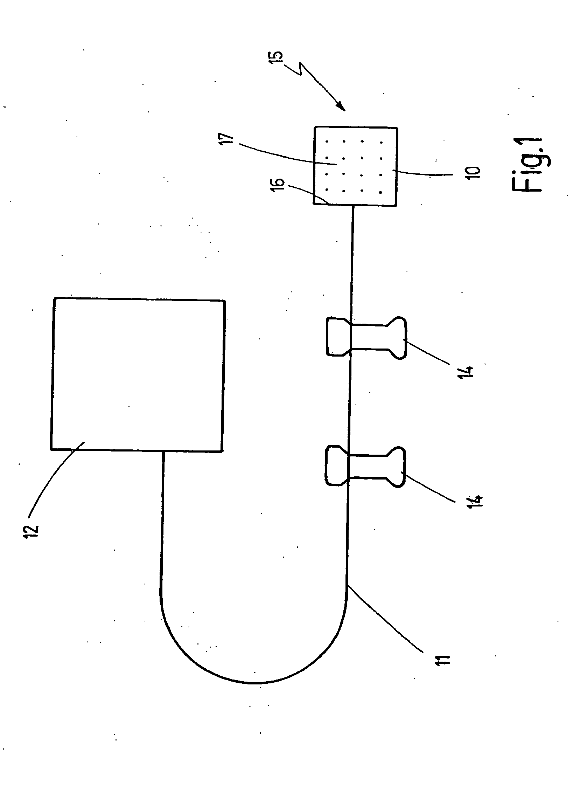

In Fig. 1 ist schematisch ein Implantat 10 dargestellt, wobei die Abmaße nicht maßstabgetreu wiedergegeben sind. Das Implantat 10 ist über ein Kabel 11 mit einer Versorgungseinheit 12 verbunden, die das Implantat 10 mit elektrischer Energie und mit Steuersignalen versorgt. An dem Kabel 11 sind Befestigungslaschen 14 vorgesehen, mit denen das Kabel am Körper der Person befestigt werden kann, der das Implantat 10 eingepflanzt wird.

Das Implantat 10 kann ein beliebiges Implantat sein, mit dem Nervenzellen angeregt werden. Im gezeigten Fall handelt es sich um ein aktives Retina-Implantat 15, das als Träger eine Folie 16 aufweist, auf der Stimulationselektroden 17 zur Abgabe vcn Stimulationssignalen an Nervenzellen angeordnet sind.

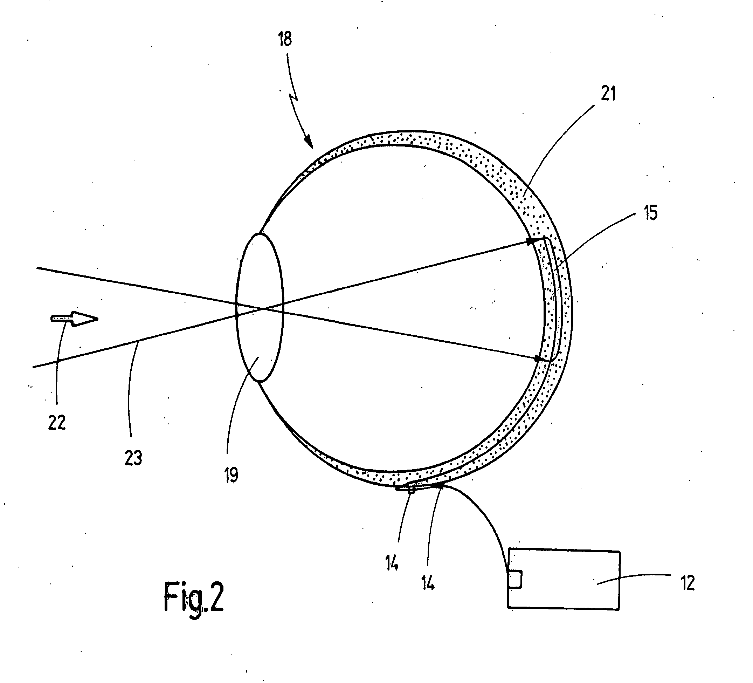

Das Retina-Implantat 15 aus Fig. 1 ist dazu bestimmt, in ein menschliches Auge 18 implantiert zu werden, das in Fig. 2 sehr schematisch dargestellt ist. Der Einfachheit halber sind nur die Linse 19 sowie die Retina 21 gezeigt, in die das Implantat 15 eingepflanzt wurde. Das Implantat 15 wird dabei vorzugsweise in den so genannten subretinalen Raum eingebracht, der sich zwischen dem Pigment-Epithel und der Fotorezeptorschicht bildet. Sofern die Fotorezeptorschicht degeneriert oder verloren ist, bildet sich der subretinale Raum zwischen dem Pigment-Epithel und der Schicht der Bipolar- und Horizontalzellen. Das Retina-Implantat 15 wird dabei so platziert, dass über die in Fig. 1 gezeigten Stimulationselektroden 17 Stimulationssignale auf Zellen in der Retina 21 ausgeübt werden können.

Durch einen Pfeil 22 angedeutetes sichtbares Licht, dessen Strahlengang bei 23 zu sehen ist, wird über die Linse 19 auf das Implantat 15 geleitet, wo das sichtbare Licht 2 in elektrische Signale umgewandelt wird, die in Stimulationssignale gewandelt werden.

Es ist zu erkennen, dass das Kabel 11 seitlich aus dem Auge herausgeführt und dort außen auf der Sclera mit den Befestigungslaschen 14 befestigt wird, bevor das Kabel weiter zu der externen Versorgungseinheit 12 führt.

Die Versorgungseinheit 12 wird dann in nicht näher gezeigter Art und Weise außerhalb des Auges beispielsweise am Schädel des Patienten befestigt. Über die Versorgungseinheit 12 wird elektrische Energie zu dem Implantat 10 gesandt, wobei gleichzeitig auch Steuersignale übermittelt werden können, die die Funktionsweise des Implantates so beeinflussen, wie dies beispielsweise in der eingangs erwähnten WO 2005/000395 A1 beschrieben ist, deren Inhalt hiermit zum Gegenstand der vorliegenden Anmeldung gemacht wird.

Es sei noch erwähnt, dass die Abmaße insbesondere des Retina-Implantates 15, der Befestigungslaschen 14 sowie der externen Versorgungseinheit 12 in den Fig. 1 und 2 weder maßstäblich noch in richtiger Größenrelation zueinander dargestellt sind.

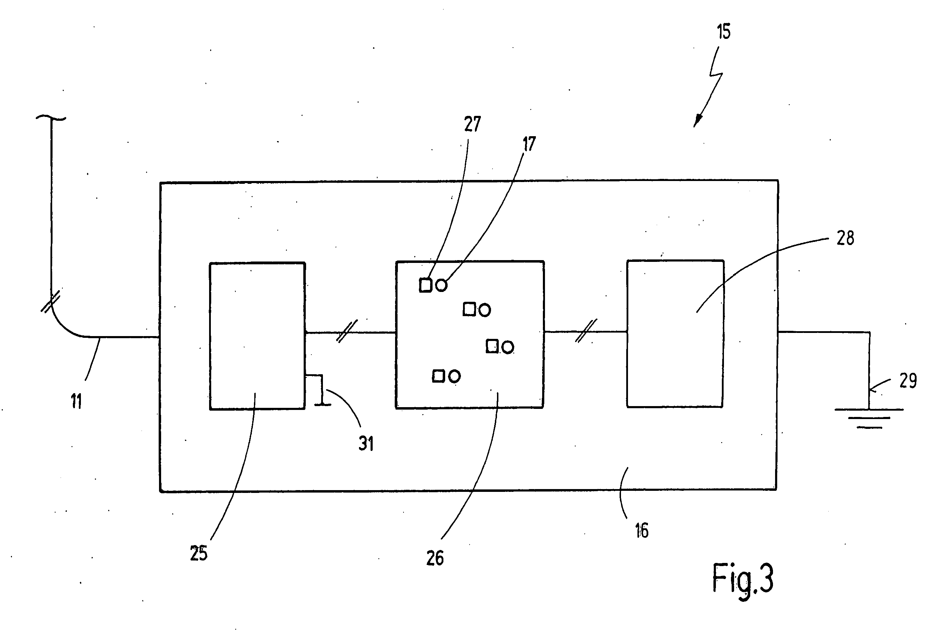

In Fig. 3 ist schematisch der Aufbau des aktiven Retina-Implantates 15 aus Fig. 1 gezeigt. Auf der Folie 16 ist zunächst eine Eingangsstufe 25 zu erkennen, der über das Kabel 11 von außen externe Fremdenergie zugeführt wird. Die Eingangsstufe 15 ist mit einer Sensoreinheit 26 verbunden, die in diesem Falle eine Vielzahl von Bildzellen 27 aufweist, die einfallendes sichtbares Licht in elektrische Signale umwandelt, die dann über die neben den jeweiligen Bildzellen angedeuteten Stimulationselektroden 17 an Nervenzellen der Retina abgegeben werden.

Die Verarbeitung der von den Bildzellen 27 erzeugten Nutzsignale erfolgt in einer Ausgangsstufe 28, die die entsprechenden Stimulationssignale erzeugt, die dann zurück zu der Sensoreinheit 26 bzw. den Stimulationselektroden 17 geführt werden.

In diesem Zusammenhang sei darauf hingewiesen, dass die Fig. 3 lediglich eine schematische, den logischen Aufbau wiedergebende Darstellung des Retina-Implantates 15 ist, die tatsächliche geometrische Anordnung der einzelnen Komponenten kann dazu führen, dass beispielsweise jede Bildzelle 27 in unmittelbarer Nachbarschaft eine Ausgangsstufe aufweist.

Das Implantat 15 ist über eine bei 29 angedeutete externe Masse mit dem Gewebe verbunden, in das das Implantat eingebracht wird. Ferner ist noch eine elektrische Masse 31 angedeutet, die in dem gezeigten Ausführungsbeispiel nicht mit der externen Masse 29 verbunden ist.

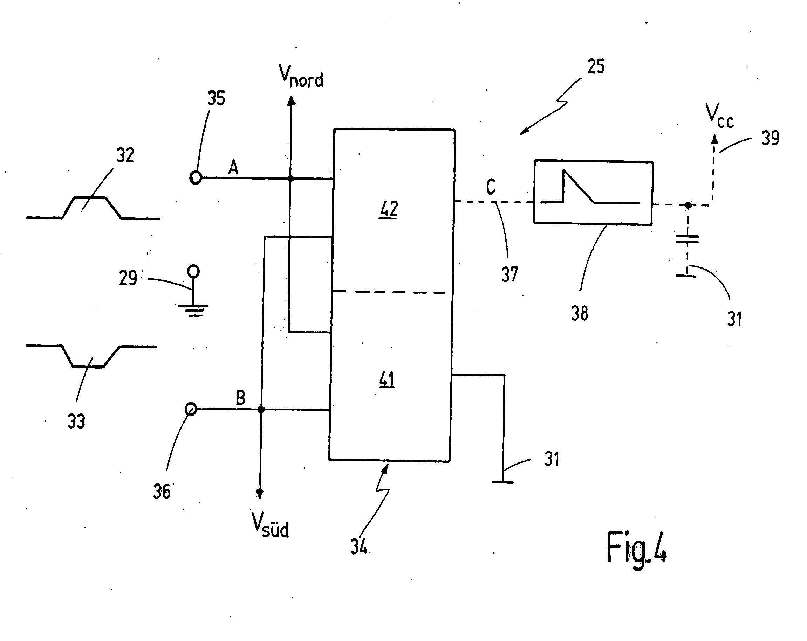

In Fig. 4 ist die Eingangsstufe 25 des Implantates 15 aus Fig. 3 im größeren Detail gezeigt.

Zunächst ist zu erkennen, dass über das Kabel zwei jeweils rechteckförmige Wechselspannungen 32, 33 zugeführt werden, die invers zueinander verlaufen, also um 180° zueinander in der Phase verschoben sind, und die symmetrisch zur externen Masse 29 liegen, so dass sie eine im zeitlichen Mittel gleichspannungsfreie Versorgung für das Implantat darstellen.

Diese beiden Wechselspannungen 32, 33 gelangen in eine Gleichrichterschaltung 24, indem sie an die beiden Eingänge 35 und 36 geführt werden. An seinem Ausgang 37 ist die Gleichrichterschaltung 34 mit einem Schalter 38 versehen, der die bei 39 angedeutete Versorgungsgleichspannung abgibt. Gestrichelt ist noch ein Kondensator gegenüber der elektrischen Masse 31 dargestellt, wobei dieser Kondensator nicht als diskretes Bauteil vorgesehen sein muss, sondern bspw. die Eingangskapazitäten der nachfolgenden integrierten Schaltungskomponenten repräsentieren kann.

Die Gleichrichterschaltung 34 weist einen ersten Ast 41 auf, der an seinem Ausgang mit der elektrischen Masse 31 verbunden ist, sowie einen zweiten Ast 42, der über den Ausgang 37 die Versorgungsgleichspannung 39 zur Verfügung stellt.

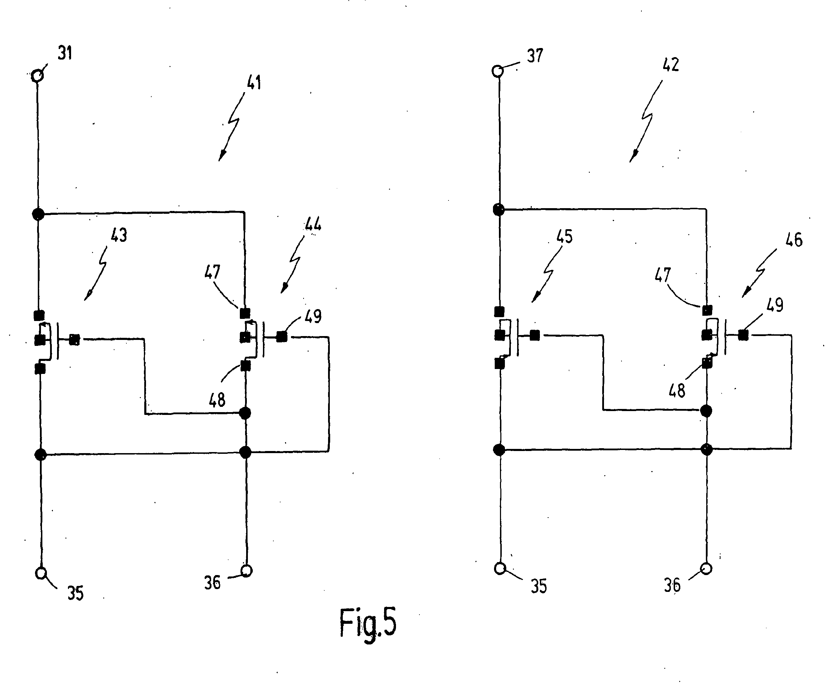

Der Aufbau der beiden Äste 41 und 42 der Gleichrichterschaltung 34 ist im Detail in Fig. 5 gezeigt.

Der erste Ast 41 weist zwei n-MOS Feldeffekttransistoren 43, 44 auf, während in dem zweiten Ast 42 zwei p-MOS Feldeffekttransistoren 45, 46 vorgesehen sind.

In jedem der beiden Äste 41 und 42 sind zwei Feldeffekttransistoren 43, 44 bzw. 45, 46 an ihrer Ausgangselektrode 47 parallel geschaltet, während an die Eingangselektroden 48 einmal die erste rechteckförmige Wechselspannung 32 und das andere Mal die zweite rechteckförmige Wechselspannung 33 geschaltet ist. An die Steuerelektrode 49 ist jeweils die mit der Eingangselektrode 48 des jeweils anderen Feldeffekttransistors in dem jeweiligen Ast verbundene Wechselspannung geführt.

Auf diese Weise werden beispielsweise die Feldeffekttransistoren 43 und 44 immer dann durchgeschaltet, wenn die Wechselspannungen 32 (am Anschluss 35) bzw. 33 (am Anschluss 36) einen negativen Puls und dementsprechend die jeweils andere Wechselspannung einen positiven Puls abgibt, der auf die Steuerelektroden 49 geleitet wird.

Mit anderen Worten bedeutet dies, dass die negativen Pulse jeweils an dem Anschluss 31 und die positiven Pulse jeweils an dem Anschluss 37 zur Verfügung stehen, so dass die Versorgungsgleichspannung an dem Anschluss 37 gegenüber dem Anschluss 31 eine Signalhöhe hat, die der Summe aus der positiven und der negativen Amplitude der Wechselspannungen entspricht. Durch die gewählte Schaltung liegt die externe Masse 29 ferner symmetrisch zwischen der elektrischen Masse 31 und der Versorgungsgleichspannung 37.

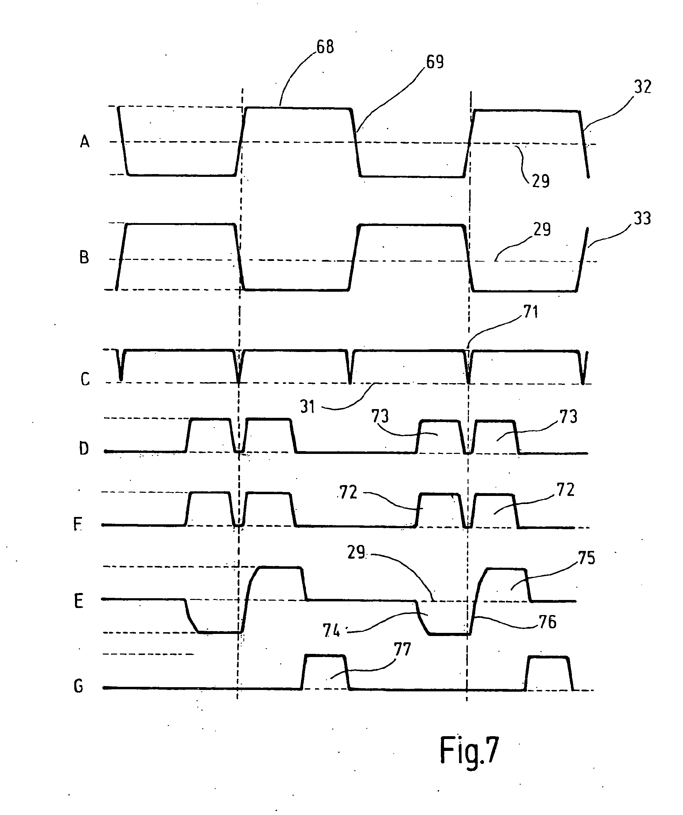

Die sich ergebenden Kurvenverläufe sind in der Fig. 7 zu erkennen, wobei bei A und B die Wechselspannungen 32 und 33 gezeigt sind, während bei C das in Fig. 3 an dem Ausgang 37 zur Verfügung stehende Signal gezeigt ist.

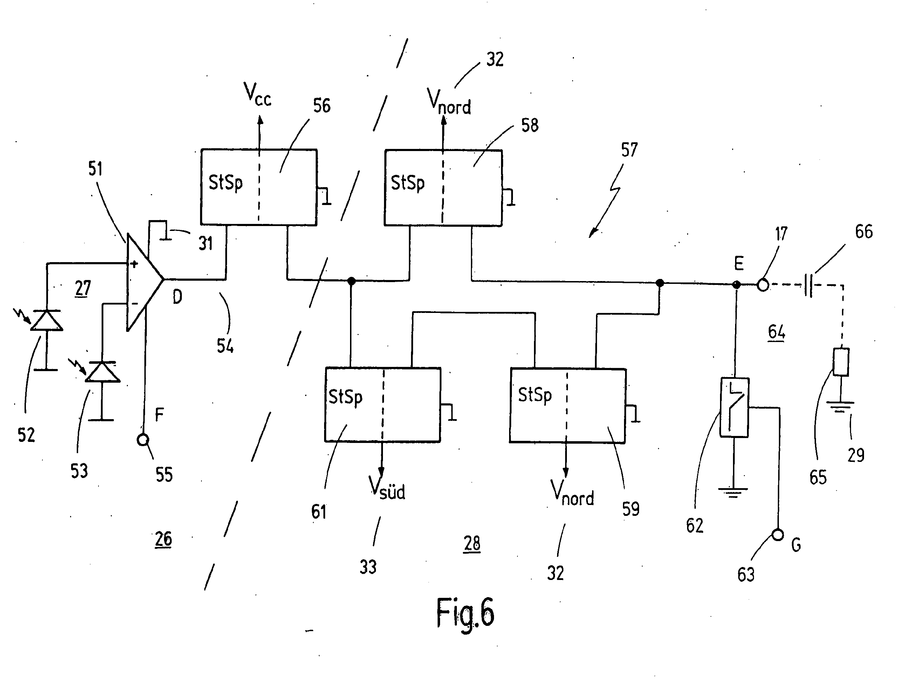

In Fig. 6 ist jetzt schematisch die Sensoreinheit 26 sowie die damit verbundene Ausgangsstufe 28 des Implantates 15 aus Fig. 3 gezeigt. Aus Gründen der Einfachheit ist in Fig. 6 lediglich eine Bildzelle 27 dargestellt, der auch nur eine einzige Ausgangsstufe 28 zugeordnet ist. In der Darstellung befindet sich die Stimulationselektrode 17 rechts neben der Ausgangsstufe 28, in der tatsächlichen geometrischen Anordnung kann die Stimulationselektrode 17 jedoch neben oder in der Bildzelle 27 angeordnet sein.

Die Bildzelle 27 weist nun an ihrem Ausgang einen Ausgangsverstärker 51 auf, der mit einer Fotodiode 52 für lokale Helligkeit und einer weiteren Fotodiode 53 für globale Helligkeit verbunden ist. Auf diese Weise gibt der Verstärker 51 an seinem Ausgang 54 ein Signal D ab, das in der Amplitude der Helligkeit des auf die lokale Fotodiode 52 gefallenen Lichtes entspricht, das jedoch über die globale Helligkeit (Fotodiode 53) nachgeregelt wurde. Der genaue Aufbau einer Bildzelle ist analog zu den in der WO 2005/000395 A1 beschriebenen Bildzellen, so dass wegen weiterer Informationen auf diese Druckschrift verwiesen werden darf.

Der Ausgangsverstärker 51 ist noch mit einem Steuersignal F an seinem Eingang 55 verbunden, über den das Ausgangssignal D an dem Ausgang 54 in noch zu beschreibender Weise getaktet werden kann.

Auf den Ausgangsverstärker 51 folgt ein Ausgangsstromspiegel 56, der den in den Ausgangsverstärker 51 fließenden Strom spiegelt und diesen also gleichzeitig auch in einen Wechselrichter 57 leitet, der in der Ausgangsstufe 28 vorgesehen ist.

Der Wechselrichter 57 weist zwei Stromspiegel 58, 59 auf, die mit der ersten Wechselspannung 32 verbunden sind und an ihrem Ausgang an die Stimulationselektrode 17 angeschlossen sind. In dem unteren Ast des Wechselrichters 57 ist noch ein weiterer Stromspiegel 61 vorgesehen, der mit der Versorgungsgleichspannung 33 verbunden ist und zwischen die Bildzelle 27 und den Stromspiegel 59 geschaltet ist.

In noch zu beschreibender Weise sorgt der so aufgebaute Wechselrichter 57 dafür, dass die Strompulse D synchron zum Phasenwechsel der Wechselspannungen 32, 33 umgepolt werden, so dass Stimulationssignale E an der Stimulationselektrode 17 zur Verfügung stehen, die im zeitlichen Mittel gleichspannungsfrei sind.

An der Stimulationselektrode 17 ist noch ein Schalter 62 angeschlossen, der über ein an dem Anschluss 63 zur Verfügung gestelltes Steuersignal G angesteuert wird und die Stimulationselektrode 17 mit der externen Masse 29 verbindet.

Schematisch dargestellt ist noch ein Gewebe 64, an das die Stimulationssignale E abgegeben werden. Als elektrisches Ersatzschaltbild ist dabei eine Reihenschaltung aus einem ohmscheh Widerstand 65 sowie einer Kapazität 66 gewählt, die die sich infolge der Elektrodenpolarisation bildende Helmholtz-Doppelschicht repräsentiert.

Das insoweit beschriebene Retina-Implantat 15 wird also zum einen im zeitlichen Mittel gleichspannungsfrei über Wechselspannungen 32, 33 versorgt, wobei es zum anderen ein ebenfalls im zeitlichen Mittel gleichspannungsfreies Anregungssignal E an umgebendes Gewebe abgibt.

Darüber hinaus benötigt das Retina-Implantat 15 noch die Steuersignale F zum Schalten des Nutzsignales D sowie das Steuersignal G zum Schalten des Stimulationssignales E. Diese Signale können zum einen auf dem Chip aus den Wechselspannungen 32 und 33 abgeleitet werden, wobei es zum anderen auch möglich ist, diese ebenfalls über das Kabel 11, nämlich in gesonderten Leitungen, zuzuführen.

Wenn die Spannungspegel dieser Steuersignale unterhalb von 0,3 V bleiben, besteht auch nicht die Gefahr der elektrolytischen Zersetzung, wobei die Steuersignale zur Stromsteuerung verwendet werden können. Dazu können die Steuersignale über eine eigene Eingangsschaltung auf dem Retina-Implantat 15 angekoppelt werden, die eine kleine Eingangsimpedanz bewirkt und einen hochohmigen Ausgang liefert, über den in unterschiedliche Lasten eingespeist werden kann. Auf diese Weise können die Steuersignale extern zugeführt werden, ohne dass die Gefahr der elektrolytischen Zersetzung besteht.

Gleiches gilt für das Signal der globalen Fotodiode 52, das in an sich bekannter Weise der Anpassung des Signales an die Umgebungshelligkeit dient. Dieses Signal für die Umgebungshelligkeit kann dabei entweder auf dem Chip selbst erzeugt werden, wie dies in der WO 2005/000395 A1 beschrieben ist, das Signal kann aber auch in der oben beschriebenen Weise extern über ein gesondertes Kabel zugeführt werden.

Der aus dem Ausgangsstromspiegel 56 aufgrund des Nutzsignales D in den Ausgangsverstärker 51 fließende Strom wird nun ebenfalls in den Wechselrichter 57 gespiegelt, wobei er entweder in den Stromspiegel 58 oder in den Stromspiegel 61 fließt, denn nur einer dieser beiden Stromspiegel ist zur Zeit mit einer Wechselspannung verbunden, die einen negativen Puls zeigt. Gegenüber der externen Masse 29 liegt die Versorgungsgleichspannung 39 (Vcc) auf +1,5 V, die elektrische Masse 31 dagegen auf -1,5 V. Wenn also die Wechselspannung 33 den negativen Puls zeigt, ist der Stromspiegel 61 auf -1,5 V gezogen, so dass der Strom aus dem Ausgangsstromspiegel 56 in den Stromspiegel 61 fließt. In diesem Stromspiegel 61 fließt gleichzeitig der gespiegelte Strom aus dem Stromspiegel 59, denn dieser ist mit der anderen Wechselspannung 32 verbunden, deren positiver Puls ebenfalls +1,5 V beträgt. Dieser Strom wird erneut gespiegelt und fließt so aus dem Stromspiegel 59 in die externe Masse 29.

Wenn sich jetzt die Polaritäten der Wechselspannungen 32, 33 ändern, fließt der gespiegelte Strom des Stromspiegels 56 in den Stromspiegel 58, da die Wechselspannung 32 jetzt auf -1,5 V liegt. In diesem Stromspiegel 58 fließt jetzt ebenfalls Strom von der externen Masse 29, so dass sich der Stromfluss durch den Kondensator 66 umgedreht hat.

Der gesamte Funktionsablauf des insoweit beschriebenen Implantates soll nun anhand der Figur 7 und unter Bezugnahme auf die Figuren 4 und 6 geschildert werden.

Wie bereits erwähnt, werden dem Implantat 15 zwei Wechselspannungen 32 und 33 zugeführt, die einen trapezförmigen Zeitverlauf aufweisen und jeweils symmetrisch zu der externen Masse 29 liegen. Die beiden Wechselspannungen 32 und 33 sind ferner invers zueinander, sie weisen also untereinander eine Phasenverschiebung von 180° auf.

Die einzelnen Spannungspulse haben eine Dachphase 68 von bspw. 20 msec sowie eine Flankenphase 69 von bspw. 1 msec. Obwohl kürzere Flankenphasen möglich sind, würden zu kurze Flankenphasen ggf. zu hochfrequenten Störungen führen, weshalb aus elektronischer Sicht eher lange Flankenphasen gewählt werden.

Nach der Gleichrichtung durch die in den Ästen 41 und 42 vorgesehenen Schalter weist die Spannung C am Ausgang 37 einen gepulsten Verlauf auf, jeweils bei einem Phasenwechsel bricht die Spannung zusammen, was durch einen Spannungseinbruch 71 dargestellt ist. Dieser Spannungseinbruch 71 entsteht durch die Verwendung der Feldeffekttransistoren 43, 44, 45, 46, während übliche Gleichrichterdioden in Brückenschaltung diesen Spannungseinbruch nicht erzwingen würden.

Prinzipiell können die in der jeweiligen Schaltung nachfolgenden Bauteile diesen Spannungseinbruch verkraften, wobei der Schalter 38 zusätzlich vorgesehen sein kann, um den Ausgang 37 während der Spannungseinbrüche 71 von der Versorgungsgleichspannung 39 zu trennen, so dass sie konstant bleiben kann.

Als nächstes folgt in Fig. 7 von oben gesehen das von dem Ausgangsverstärker 51 abgegebene Nutzsignal D. Die Bildzelle 27 erzeugt zunächst einmal einen langen Puls, dessen Amplitude der Intensität des auf die Fotodiode 52 gefallenen Lichtes entspricht.

Dieses Ausgangssignal wird nun jedoch nicht als ein durchgehender Puls abgegeben, vielmehr wird der Verstärker 51 über das Steuersignal F getaktet, das zwei symmetrisch zu den Flanken 59 verlaufende Steuerpulse 72 aufweist.

Auf diese Weise wird auch das Nutzsignal D in zwei Spannungspulse 73 aufgeteilt, die symmetrisch zu den Flanken der Wechselspannungen 32, 33 liegen.

Durch den Wechselrichter 57 wird jetzt der zeitlich erste der beiden Pulse 73 gespiegelt, so dass sich das Stimulationssignal E ergibt, das einen gegenüber der externen Masse 29 kathodischen Vorbereitungspuls 74 sowie einen gegenüber der externen Masse 29 anodischen Anregungspuls 75 umfasst. Das Stimulationssignal E stellt also eine biphasische Anregung für das umgebende Gewebe 64 dar, wobei die Anregung mit einem kathodischen Puls beginnt. Auf diese Weise steht eine größere Flanke 76 für das Anregungssignal zur Verfügung, was zu einem kurzfristigen hohen Stromfluss in das umgebende Gewebe 64 hinein führt, so dass eine gute Anregung der nachfolgenden Nervenzellen erreicht wird.

Um jetzt dafür zu sorgen, dass im Mittel keine Ladungsverschiebung in das Gewebe 64 hinein erfolgt, wird nach Abschluss der Pulsfolge 74, 75 der Schalter 62 über das Steuersignal G kurzfristig geschlossen, so dass sich der Kondensator 66 entlädt. Dabei ist es wichtig, dass der Schalter 62 mit der externen Masse 29 verbindet.

Auf diese Weise ist also zum einen das Stimulationssignal E im zeitlichen Mittel gleichspannungsfrei, wobei zum anderen ebenfalls dafür gesorgt wird, dass sämtliche in das Gewebe hineingeflossene Ladung zurückgeführt wird, so dass mit dem nächsten folgenden Nutzsignal D erneut eine Anregung vorgenommen werden kann.

## Patentansprüche

1. Vorrichtung mit einer Versorgungseinheit (12) und einem mit der Versorgungseinheit (12) verbundenen, implantierbaren Implantat (10), das dazu dient, über zumindest eine Stimulationselektrode (17) elektrische Stimulationssignale (E) an umgebendes Gewebe (64) abzugeben, wobei das Implantat (10) über seine mit der Versorgungseinheit (12) verbundene Eingangsstufe (25) mit elektrischer Energie versorgt wird,
**dadurch gekennzeichnet, dass** die Versorgungseinheit (12) mit dem Implantat (10) über Kabel (11) verbunden ist, über die sie die Eingangsstufe (25) mit zumindest zwei im Wesentlichen rechteckförmigen elektrischen Wechselspannungen (32, 33) versorgt, die gegenüber einer mit dem Gewebe (64) verbindbaren externen Masse (29) im zeitlichen Mittel zumindest nahezu gleichspannungsfrei sind, wobei die Wechselspannungen (32, 33) zueinander in der Phase verschoben sind.

2. Vorrichtung nach Anspruch 1, **dadurch gekennzeichnet, dass** die Eingangsstufe (25) eine Gleichrichterschaltung (34) umfasst, die die Wechselspannungen (32, 33) gleichrichtet und zu einer Versorgungsgleichspannung (39) addiert.

3. Vorrichtung nach Anspruch 1 oder 2, **dadurch gekennzeichnet, dass** die Eingangsstufe (25) mit einer ersten und einer zweiten Wechselspannung (32, 33) versorgt wird, die bei etwa gleichem Kurvenverlauf und etwa gleichen Amplituden zumindest nahezu invers zueinander verlaufen.

4. Vorrichtung nach Anspruch 2 und 3, **dadurch gekennzeichnet, dass** die Gleichrichterschaltung (34) aus der ersten und der zweiten Wechselspannung (32, 33) die Versorgungsgleichspannung (39) derart erzeugt, dass ihre elektrische Masse (31) und die Versorgungsgleichspannung (39) symmetrisch zu der externen Masse (29) liegen.

5. Vorrichtung nach Anspruch 4, **dadurch gekennzeichnet, dass** die Gleichrichterschaltung (34) Schalter (43, 44, 45, 46) für die jeweilige Wechselspannung (32, 33) aufweist, die über die jeweils andere Wechselspannung (33, 32) angesteuert werden.

6. Vorrichtung nach Anspruch 5, **dadurch gekennzeichnet, dass** die Gleichrichterschaltung (34) zwei Äste (41, 42) mit jeweils zwei an ihren Ausgangselektroden (47) parallel geschalteten Feldeffekttransistoren (43, 44, 45, 46) umfasst, wobei die Ausgangselektroden (47) in dem ersten Ast (41) die elektrische Masse (31) bilden und die Ausgangselektroden (47) in dem zweiten Ast (42) die Versorgungsgleichspannung (39) abgeben, und wobei jeder Feldeffekttransistor (43, 44, 45, 46) an seiner Eingangselektrode (48) mit einer der beiden Wechselspannungen (32, 33) und an seiner Steuerelektrode (49) mit der anderen der beiden Wechselspannungen (33, 32) verbunden ist.

7. Vorrichtung nach Anspruch 6, **dadurch gekennzeichnet, dass** der Ausgang (37) des zweiten Astes (42) über einen Schalter (38) mit der Versorgungsgleichspannung (39) verbunden ist, der immer dann geöffnet ist, wenn die Wechselspannungen (32, 33) ihre Polarität gegenüber der externen Masse (29) ändern.

8. Vorrichtung nach einem der Ansprüche 1 bis 7, **dadurch gekennzeichnet, dass** jede Wechselspannung (32, 33) einen trapezförmigen Spannungsverlauf aufweist, mit Dachphasen (68) konstanter Spannung und kurzen Flankenphasen (69), in denen die Polarität gegenüber der externen Masse (29) wechselt, wobei die Länge der Dachphasen (68) im Bereich von höchstens 100 msec liegt, und die Flankenphasen (69) vorzugsweise höchstens 10 %, vorzugsweise weniger als 1 % aber mehr als 0,05% der Dachphase (68) betragen.

9. Vorrichtung nach einem der Ansprüche 1 bis 8, **dadurch gekennzeichnet, dass** das Implantat (10) eine Sensoreinheit (26), die aus extern zugeführten Signalen ein Nutzsignal (D) erzeugt, und eine Ausgangsstufe (28) umfasst, die aus dem Nutzsignal (D) die Stimulationssignale (E) erzeugt.

10. Vorrichtung nach Anspruch 9, **dadurch gekennzeichnet, dass** das Implantat (10) ein aktives Retina-Implantat (15) ist, dessen Sensoreinheit (26) eine Vielzahl von Bildzellen (27) umfasst, die einfallendes Licht in elektrische Signale umwandeln, wobei die Sensoreinheit (26) aus den elektrischen Signalen das Nutzsignal (D) in Form von analogen Spannungspulsen (73) bestimmter Pulslänge und bestimmter Pulsabstände erzeugt, deren Amplituden von der jeweiligen Intensität des einfallenden Lichts abhängen.

11. Vorrichtung nach Anspruch 9 oder 10, **dadurch gekennzeichnet, dass** die Ausgangsstufe (28) die Stimulationssignale (E) im zeitlichen Mittel im Wesentlichen gleichspannungsfrei an umgebendes Gewebe (64) abgibt, wozu sie vorzugsweise einen Wechselrichter (57) umfasst.

12. Vorrichtung nach Anspruch 11, **dadurch gekennzeichnet, dass** der Wechselrichter (57) die Spannungspulse (73) synchron zu den Wechselspannungen (32, 33) in ihrer Polarität gegenüber der externen Masse (29) umpolt.

13. Vorrichtung nach Anspruch 12, **dadurch gekennzeichnet, dass** der Wechselrichter (57) zwei parallel zueinander zwischen den Ausgang (54) der Sensoreinheit (26) und die Stimulationselektrode (17) geschaltete Äste aufweist, die beide an ihrem Ausgang je einen mit der ersten Wechselspannung (32) verbundenen ersten Stromspiegel (58, 59) umfassen, wobei der eine Ast einen mit der zweiten Wechselspannung (33) verbundenen zweiten Stromspiegel (61) umfasst, der zwischen die Sensoreinheit (26) und den ersten Stromspiegel (59) geschaltet ist.

14. Vorrichtung nach einem der Ansprüche 9 bis 13, **dadurch gekennzeichnet, dass** die Sensoreinheit (26) an ihrem Ausgang (54) einen Ausgangsverstärker (51) aufweist, dessen Ausgang über ein Steuersignal (F) immer dann abgeschaltet ist, wenn die Wechselspannungen (32, 33) ihre Polarität gegenüber der externen Masse (29) ändern.

15. Vorrichtung nach einem der Ansprüche 11 bis 14, **dadurch gekennzeichnet, dass** der Wechselrichter (57) an seinem Ausgang einen Schalter (62) aufweist, der den Ausgang immer dann kurzfristig mit der externen Masse (29) verbindet, wenn ein Spannungspuls (73) beendet ist, dessen Amplitude also für die Dauer des Pulsabstandes auf die interne Masse (31) geschaltet wird.

## Claims

1. Device comprising a supply unit (12) and an implantable implant (10) connected to said supply unit (12) and used to emit electrical stimulation signals (E) to surrounding tissue (64) by means of at least one stimulation electrode (17), whereby the implant (10) is supplied with electrical energy via its input stage (25), which input stage is connected to said supply unit (12),
**characterized in that** the supply unit (12) is connected to the implant (10) by a cable (11), by means of which it supplies the input stage (25) with at least two substantially square electrical AC voltages (32, 33), which are, averaged over time, at least virtually free of a DC voltage with respect to an external ground (29), which external ground can be connected to the tissue (64), with the AC voltages (32, 33) being phase-shifted with respect to one another.

2. Device according to Claim 1, **characterized in that** the input stage (25) comprises a rectifier circuit (34), which rectifies the AC voltages (32, 33) and adds them to form a DC supply voltage (39).

3. Device according to Claim 1 or 2, **characterized in that** the input stage (25) is supplied with a first and a second AC voltage (32, 33) which, while exhibiting approximately the same curve profile and approximately the same amplitudes, are at least virtually inverted with respect to one another.

4. Device according to Claims 2 and 3, **characterized in that** the rectifier circuit (34) generates the DC supply voltage (39) from the first and the second AC voltages (32, 33) such that the electrical ground (31) thereof and the DC supply voltage (39) are symmetric with respect to the external ground (29).

5. Device according to Claim 4, **characterized in that** the rectifier circuit (34) has switches (43, 44, 45, 46) for the respective AC voltage (32, 33) which are actuated by the respective other AC voltage (33, 32).

6. Device according to Claim 5, **characterized in that** the rectifier circuit (34) comprises two branches (41, 42) with respectively two field-effect transistors (43, 44, 45, 46) connected in parallel at their output electrodes (47), with the output electrodes (47) in the first branch (41) forming the electrical ground (31) and the output electrodes (47) in the second branch (42) emitting the DC supply voltage (39), and with every field-effect transistor (43, 44, 45, 46) being connected to one of the two AC voltages (32, 33) at its input electrode (48) and being connected to the other one of the two AC voltages (33, 32) at its control electrode (49).

7. Device according to Claim 6, **characterized in that** the output (37) of the second branch (42) is connected to the DC supply voltage (39) via a switch (38) which is always open when the AC voltages (32, 33) change their polarity with respect to the external ground (29).

8. Device according to anyone of Claims 1 to 7, **characterized in that** every AC voltage (32, 33) has a trapezoidal voltage profile with top phases (68) of constant voltage and short pulse-edge phases (69) in which the polarity with respect to the external ground (29) changes, with the duration of the top phases (68) being of the order of at most 100 ms, and the pulse-edge phases (69) preferably being at most 10%, preferably being less than 1% but more than 0.05% of the top phase (68).

9. Device according to anyone of Claims 1 to 8, **characterized in that** it comprises a sensor unit (26), which generates a useful signal (D) from externally fed signals, and an output stage (28) which generates the stimulation signals (E) from the useful signal (D).

10. Device according to Claim 9, **characterized in that** the implant (10) is an active retinal implant (15), the sensor unit (26) of which comprising a multiplicity of image cells (27) which convert incident light into electrical signals, with the sensor unit (26) using the electrical signals to generate the useful signal (D) in the form of analogue voltage pulses (73) of a certain pulse duration and certain pulse spacing, the amplitudes of which depending on the respective intensity of the incident light.

11. Device according to Claim 9 or 10, **characterized in that** the output stage (28) emits the stimulation signals (E) to the surrounding tissue (64) in, averaged over time, a substantially DC voltage free fashion and, for this purpose, it preferably comprises an inverter (57).

12. Device according to Claim 11, **characterized in that** the inverter (57) reverses the polarity of the voltage pulses (73) with respect to the external ground (29) synchronously with the AC voltages (32, 33).

13. Device according to Claim 12, **characterized in that** the inverter (57) has two branches, connected in parallel to one another, between the output (54) of the sensor unit (26) and the stimulation electrode (17), both branches respectively comprising at their output a first current mirror (58, 59) connected to the first AC voltage (32) and with the one branch comprising a second current mirror (61), connected to the second AC voltage (33), which second current mirror (61) is connected between the sensor unit (26) and the first current mirror (59).

14. Device according to anyone of Claims 9 to 13, **characterized in that** the sensor unit (26) has an output amplifier (51) at its output (54), the output of which is always switched off by means of a control signal (F) when the AC voltages (32, 33) change their polarity with respect to the external ground (29).

15. Device according to anyone of Claims 11 to 14, **characterized in that** the inverter (57) has a switch (62) at its output, which always briefly connects the output to the external ground (29) when a voltage pulse (73) is over, the amplitude of which thus being connected to the internal ground (31) for the duration of the pulse spacing.

## Revendications

1. Dispositif comportant une unité d'alimentation (12) et un implant (10) implantable relié à l'unité d'alimentation (12) qui sert en outre à délivrer par le biais d'au moins une électrode de stimulation (17) des signaux de stimulation électrique (E) à un tissu environnant (64), l'implant (10) étant alimenté en énergie électrique par un palier d'entrée (25) reliée à l'unité d'alimentation (12),
**caractérisé en ce que** l'unité d'alimentation (12) est reliée à l'implant (10) par un câble (11) par lequel elle alimente le palier d'entrée (25) avec au moins deux tensions alternatives (32, 33) essentiellement rectangulaires qui sont au moins à peu près dépourvues de tension continue en moyenne temporelle par rapport à une masse externe (29) pouvant être reliée au tissu (64), les tensions alternatives (32, 33) étant décalées l'une par rapport à l'autre dans la phase.

2. Dispositif selon la revendication 1, **caractérisé en ce que** le palier d'entrée (25) comprend un circuit redresseur (34) qui redresse les tensions alternatives (32, 33) et les ajoute en une tension continue d'alimentation (39).

3. Dispositif selon la revendication 1 ou 2, **caractérisé en ce que** le palier d'entrée (25) est alimenté avec une première et une deuxième tension alternative (32, 33) qui évoluent pratiquement inversement l'une par rapport à l'autre sur à peu près la même courbe et à peu près aux mêmes amplitudes.

4. Dispositif selon les revendications 2 et 3, **caractérisé en ce que** le circuit redresseur (34) génère à partir des première et deuxième tensions alternatives (32, 33) la tension continue d'alimentation (39) de telle sorte que leur masse électrique (31) et la tension continue d'alimentation (39) soient symétriques à la masse externe (29).

5. Dispositif selon la revendication 4, **caractérisé en ce que** le circuit redresseur (34) présente des commutateurs (43, 44, 45, 46) pour la tension alternative respective (32, 33) qui sont commandés par l'autre tension alternative respective (33, 32).

6. Dispositif selon la revendication 5, **caractérisé en ce que** le circuit redresseur (34) comprend deux branches (41, 42) dotées respectivement de deux transistors à effet de champ (43, 44, 45, 46) montés en parallèle sur leurs électrodes de sortie (47), les électrodes de sortie (47) formant dans la première branche (41) la masse électrique (31) et les électrodes de sortie (47) délivrant dans la deuxième branche (42) la tension continue d'alimentation (39) et chaque transistor à effet de champ (43, 44, 45, 46) étant relié sur son électrode de sortie (48) avec l'une des deux tensions alternatives (32, 33) et sur son électrode de commande (49) avec l'autre des deux tensions alternatives (33, 32).

7. Dispositif selon la revendication 6, **caractérisé en ce que** la sortie (37) de la deuxième branche (42) est reliée par un commutateur (38) avec la tension continue d'alimentation (39) qui est toujours ouverte lorsque les tensions alternatives (32, 33) modifient leur polarité par rapport à la masse externe (29).

8. Dispositif selon l'une des revendications 1 à 7, **caractérisé en ce que** chaque tension alternative (32, 33) présente une variation de tension trapézoïdale avec des phases de plateau (68) de tension constante et des phases de flanc (69) courtes dans lesquelles la polarité par rapport à la masse externe (29) change, la longueur des phases de plateau (68) se situant dans la plage d'au plus 100 msec et les phases de flanc (69) étant de préférence au plus de 10 %, de préférence inférieures à 1 % mais supérieures à 0,05 % de la phase de plateau (68).

9. Dispositif selon l'une des revendications 1 à 8, **caractérisé en ce que** l'implant (10) comprend une unité de capteur (26) qui génère un signal utile (D) à partir des signaux acheminés de l'extérieur et un palier de sortie (28) qui génère à partir du signal utile (D) les signaux de stimulation (E).

10. Dispositif selon la revendication 9, **caractérisé en ce que** l'implant (10) est un implant rétinien actif (15) dont l'unité de capteur (26) comprend de multiples cellules d'image (27) qui convertissent la lumière incidente en signaux électriques, l'unité de capteur (26) générant le signal utile (D) à partir des signaux électriques sous la forme d'impulsions de tension analogiques (73) de longueurs d'impulsion déterminées et à intervalles d'impulsions déterminés dont les amplitudes dépendent de l'intensité respective de la lumière incidente.

11. Dispositif selon la revendication 9 ou 10, **caractérisé en ce que** le palier de sortie (28) diffuse les signaux de stimulation (E) en moyenne temporelle essentiellement sans tension continue sur le tissu environnant (64), en comprenant à cette fin de préférence un onduleur (57).

12. Dispositif selon la revendication 11, **caractérisé en ce que** l'onduleur (57) repolarise les impulsions de tension (73) de façon synchrone avec les tensions alternatives (32, 33) en leur polarité par rapport à la masse externe (29).

13. Dispositif selon la revendication 12, **caractérisé en ce que** l'onduleur (57) présente deux branches montées en parallèle l'une par rapport à l'autre entre la sortie (54) de l'unité de capteur (26) et l'électrode de stimulation (17) qui comprennent toutes les deux à leur sortie, respectivement un premier courant en rapport géométrique (58, 59) relié à la première tension alternative (32), la branche comprenant un deuxième courant en rapport géométrique (61) relié à la deuxième tension alternative (33), qui est monté entre l'unité de capteur (26) et le premier courant en rapport géométrique (59).

14. Dispositif selon l'une des revendications 9 à 13, **caractérisé en ce que** l'unité de capteur (26) présente à sa sortie (54) un amplificateur de sortie (51) dont la sortie est mise hors circuit par un signal de commande (F) lorsque les tensions alternatives (32, 33) modifient leur polarité par rapport à la masse externe (29).

15. Dispositif selon l'une des revendications 11 à 14, **caractérisé en ce que** l'onduleur (57) présente sur sa sortie un commutateur (62) qui relie la sortie à court terme avec la masse externe (29) lorsqu'une impulsion de tension (73) est achevée, dont l'amplitude est commutée également pour la durée de l'intervalle d'impulsion sur la masse interne (31).
